Europäisches Patentamt

European Patent Office

Office européen des brevets

(11) Publication number: **0 389 376 B1**

# EUROPEAN PATENT SPECIFICATION

(45) Date of publication of patent specification: **07.06.95**   (51) Int. Cl.6: **C07K 16/00**, A61K 51/00

(21) Application number: **90400794.5**

(22) Date of filing: **22.03.90**

The file contains technical information submitted after the application was filed and not included in this specification

(54) **Production and characteristics of anti-myosin mouse monoclonal antibody.**

(30) Priority: **23.03.89 US 327747**

(43) Date of publication of application:
**26.09.90 Bulletin 90/39**

(45) Publication of the grant of the patent:
**07.06.95 Bulletin 95/23**

(84) Designated Contracting States:
**AT CH DE FR GB IT LI NL SE**

(56) References cited:
**EP-A- 0 163 041**

**JOURNAL OF BIOLOGICAL CHEMISTRY, vol. 257, no. 4, 25 February 1982, US; R.A. CHIZZONITE et al., pp. 2056-2065&NUM;**

**AMERICAN JOURNAL PHYSIOL., vol. 255, 1988, American Physiological Society; K.A. ESSER et al., pp. H659-H663&NUM;**

(73) Proprietor: **BIOTECH CARDIO-VISION
8480 St. Laurent Blvd.
Montreal, Ouebec H2P 2M6 (CA)**

(72) Inventor: **Sikorska, Hanna
15724 Pierre-Legault
Pierrefonds, PO, H9H 9Z7 (CA)**
Inventor: **Savoie, Sylvine
4990 Edouard-Montpetit Blvd
Montreal, PO, H3W 1P9 (CA)**
Inventor: **Desputeau, Clémence
902 Royale St.
Bellefeuille, PO, J0R 1A0 (CA)**

(74) Representative: **Peaucelle, Chantal et al
Cabinet Armengaud Aine
3, avenue Bugeaud
F-75116 Paris (FR)**

## Description

BACKGROUND OF THE INVENTION

One of the most interesting aspects in modern cell biology is the mechanism by which cells and tissues respond to various kinds of stress. For the heart this response is especially important, because the changing demands induce a protection which is essential for survival. A temporary stimulation of the heart is regulated by changes in diastolic volume (preload), aortic pressure (afterload), heart rate, adrenergic mechanism, and substrate availability. If the increase in demand is chronic the response leads to the development of adaptional factors. Two processes are of importance: i) hypertrophy due to an increase in the size of the myocytes and ii) an elevated efficiency of the concentration of each sarcomere. The restructuring of the different components cell depends on the nature, duration, and intensity of the stress, as well as on the age and the species (Alpert, N.R., L.A. Mulieri: Med. Sci. Sports Exerc. 18, 309-313 (1986)). This is particularly true for muscle cells. Muscles are classified broadly into the two groups of striated muscles and smooth muscles. Striated muscles are further classified into cardiac muscles and skeletal muscles, the skeletal muscles being further classified into fast muscles and slow muscles. It has been reported that these can be distinguished immunochemically through the difference in immunogenicity of the myosin molecules which are major constituents of muscles (Masaki et al., J. Biochem., 76, 441, (1974)). At least two molecular variants of myosin heavy chains, coded by distinctive genes (Mahdavi V, Chambers VAP, Nadal-Ginard B: Proc. Natl. Acad. Sci. USA. 81, 2626-3630 (1984)), have been described in the human myocardium: an atrial HCα - and a ventricular HCβ-type (Gorza, L., J.J. Mercadier, K. Schwartz, L.E. Thornell, S. Satore, S. Schiaffino: Circ. Res. 54, 694-702 (1984); Mercadier, J.J., P. Bouveret, L. Gorza, S. Schiaffino, W.A. Clark, R. Zak, B. Swynghedauw, J. Schwartz: Circ. Res. 53, 52-63 (1983); Hirzel HO, Tuchschmid CR, Schneider J, Krayenbuehl HP, Schaub MC: Circ. Res. 57 729-740 (1985); Kuro-O M, Tsuchimochi H, Uedas, Takaku F, Yazaki Y: J. Clin Invest. 77, 340-347 (1986); Tsuchimochi H, Kuro-O M, Takaku F, Yoshida K, Kawana M, Kimata S, Yazaki Y.: Jap Circ. J. 50, 1044-1052 (1986); Dechesne C, et al., J. Mol. Cell Cardiol. 17, 753-767 (1985); Bouvagnet P, et al. Circ. Res. 55, 794-804 (1984); Tsuchimochi H, et al.: J. Clin. Invest. 81, 110-118, (1988).

They differ in both ATPase activity and mobility in pyrophosphate polyacrylamide gel electrophoresis. Hcα has a higher $Ca^{++}$- and actin-activated ATP-ase activity than does HCβ and migrates faster in gels (Yasaki et al., Circ. Res. 35, 15, 1974; Hoh et al., J. Mol. Cell. Cardiol. 10, 1053-1076, 1978).

These two types of heavy chains have been shown in animals to form myosin molecules composed either of an αα homodimer or an αβ-heterodimer , or a ββ-homodimer which correspond to the V-1, V-2 and V-3 isoforms described by Hoh and coworkers (Hoh JFY: J. Mol. Cell Cardiol. 10, 1053-1076, 1978). V-1 exhibits higher adenosine triphosphatase (ATPase) activity than V-3.

The ratio of these myosin isoforms varies according to the physiological and pathological state or developmental stage of the myocardium (for reviews, see Mercadier, et al.: Circ. Res: 53, 52-62, 1983; Tobacman et al: J. Biol. Chem., 259, 11226-11230, 1984). The change from V-1 toward V-3 is accompanied by a decrease in ATPase activity and speed of contraction (Schwartz et al.: J. Mol. Cell Cardiol., 13, 1071-1073, 1981; Ebrecht et al.: Basic Res. Cardiol., 77, 220-234, 1982), an improved economy of force generation (Alpert et al.: Fed. Proc. 41, 192-198, 1981; Alpert et al.: Circ. Res., 50, 491-500, 1982), and decreased oxygen consumption (Kissling et al.: Basic Res. Cardiol. 77, 255-270, 1982). These changes in myosin HC composition found in animals were interpreted as an adaptation of the myocardial cell, together with compensatory hypertrophy of the muscle, to new functional requirements.

In man, normal ventricular tissue contains predominantly the V-3 species (ββ-homodimer) and only few amounts (0-15%) of the V-1 species (α,α-homodimer). The opposite is true for the human atrium, where the abundant myosin isoform is V-1 and, to a lesser degree, isozyme V-3.

Human fetal atrium is composed mostly of α-HC during the first 23 weeks of gestation. (Bouvagnet et al.: Circ. Res. 61, 329-336, 1987). β-HC is already expressed as traces at 14 weeks of gestation, and its expression increase progressively until birth, resulting in a great augmentation in β-HC. During this course, β-HC always predominates in certain areas (the crista terminalis and the interatrial septum) but not in other areas (the auricles). Preceding birth, the fetal ventricle is composed mostly of β-HC. From 14 weeks of gestation to birth, α-HC is expressed in very rare fibers. Then, after birth, a large number of fibers simultaneously synthesize α-HC.

Animal studies have shown evidence of thyroid hormone affecting the expression of isomyosin (for review, see Swynghedauw B.: Physiol. Rev., 66, 710-771, 1986). It is not yet known whether the human heart has the same property, but at birth, increase of β-HC expression in the atrium and α-HC in the ventricle, is associated with the rapid rise in circulating thyroid hormone levels. Since Chizzonite and Zak (J.

Biol. Chem., 259, 12628-12632, 1984) have clearly demonstrated the role of thyroid hormone in the induction of α-HC expression in neonatal rat ventricle, thyroid hormone hypothetically could also induce ventricular α-HC expression in humans. Thyroid hormone has highly tissue specific effect and can switch HC gene expression on or off depending on the tissue where it is expressed (Izumo, et al.: Science, 231, 597-600, 1986). Insulin also is involved in the regulation of myosin HC expression (Dillman, et al.: J. Biol. Chem. 259, 2035-2038, 1984).

In pressure-overloaded human atrial muscle, there is a transition from α- to β- HC (Kurabayashi, et al.: J. Clin. Invest., 82, 524-531, 1988; Tsuchimochi, et al.: J. Clin. Invest., 74, 662-665, 1984; Buttrick, et at.: Circulation, 74, 477-483, 1986; Schlesinger, et al.: Biochem. Intern. 11, 747-753, 1985) as an early adaptation to the imposed load.

Myocardial infarction and associated work overload cause a transition in the light chain complements of the myosin (Hoffman, et al.: Basic Res. Cardiol. 32, 359-369, 1987; Hoffman et al.: Biomed. Biochem. Acta, 46, S724-S727, 1987). Ventricular myosin light chains are found in pressure overloaded atria and atrial light chains have also been identified in the infarcted ventricle of the human heart. The relative proportions of atrial myosin heavy chains are changed after infarction. A decrease in β-HC and a corresponding increase in α-HC were observed. Ventricular hypertrophy in patients with coronary insufficiency induces α-HC expression. The relative part of this myosin type amounts to 20%.

In the field of muscle research, antibodies against muscle proteins have long been utilized.

In recent years, as a method for obtaining an antibody having high specificity in a large amount, it has been known to prepare a hybridoma, by fusion of an antibody-producing cell with a myeloma cell and culturing the hybridoma thus obtained to produce a monoclonal antibody (Kohler et al., Nature, Vol. 256, p. 495 (1975)), and a large number of monoclonal antibodies have been obtained by such a method.

Further, these antibodies can be labelled with radioisotopes and used for localization of myocardial infarction.

There is provided in USP 4,767,843 (Yazaki et al.) a monoclonal antibody which has specificity to cardiac myosin heavy chain α type but does not recognize cardiac myosin heavy chain β type and also a monoclonal antibody which has specificity to cardiac myosin heavy chain β type but does not recognize cardiac myosin heavy chain α type.

In view of the above presented evidence, it would be highly desirable to have a monodonal antibody which recognizes α- and β-heavy chain of atrial and ventricular human myosin for imaging of myocardial infarction, since a single antibody molecule would demarcate the infarcted zone regardless of the patient's age the pathological or the physiological condition.

Further, it would also be useful to have a monoclonal antibody which could provide the diagnosis of both atrial and ventricular myocardial infarction simultaneously.

## SUMMARY OF THE INVENTION

In accordance with the present invention there is provided a monoclonal antibody which recognizes α- and β- heavy chain of atrial and ventricular human myosin and β-heavy chain of slow human skeletal muscle.

Further, the antibody of the present invention can be labelled with radioisotopes such as technetium-99 m, indium, iodine, etc., and applied for immunodetection, in which it is measured by whole-body gamma scintigraphy after administration into a patient, whereby localization of myocardial infarction is rendered possible.

More specifically, the present invention provides a monoclonal antibody which enables the diagnosis of both atrial and ventricular myocardial cell death.

The currently available techniques for diagnosis of myocardial infarction (MI) are not accurate enough to make a precise distinction between the necrotic and ischemic damage to myocardium. The adequate diagnosis is, however, essential for successful immediate treatment and longterm survival of patients. The present study intends to develop a new parameter that could assist the cardiologist in making the right diagnosis. The employed method involves the imaging of myosin heavy chains within the heart tissue using specific mouse monoclonal antibody tagged with radioisotope. This method is based on the principle that the cell death is caused by loss of membrane integrity. Intracellular enzymes leak to extracellular fluid and intracellular protein myosin is exposed to extracellular markers that are normally excluded from alive cells. Thus myosin becomes available to react with labelled antibodies or antibody fragments.

Myosin, the major muscle contractile protein, consists of two heavy chairs each of about 200,000 daltons and two pairs of light chains. Heavy chains form an α-helical coiled tail over a length of light chains. In ventricular myocardium light chain 1 is 27,000 d and light chain 2 20,000 d. The intact molecule has a

3

molecular weight of 500,000 daltons and due to heavy chains is soluble only in high salt solutions (>0.3 M KCl). The catalytic and actin binding properties of myosin reside in the water-soluble heads. Both heavy and light chains of myosin derived from different species and tissues are structurally, enzymatically and antigenetically distinct.

Multiple molecular forms of myosin heavy chains have been demonstrated in fast, slow and embryonic skeletal muscle as well as in atria and ventricles of the myocardium. A single type of muscle may contain more than one molecular form of myosin heavy chain. Two isozymes, one being $V_1$ ($\alpha$-chain) having a high ATPase activity and the other being $V_3$ ($\beta$-type) having a low ATPase activity exist in cardiac muscles. In humans, atrial muscles contain primarily $\alpha$-type heavy chain while ventricular muscles contain substantially $\beta$-type chain.

This is a great improvement over the previous monoclonal antibodies, in that it enables the imaging of both the a trial and ventricular region by using only one antibody, and that it will image the regions of the infarcted myocardium where transitions of myosin isozymes had occured due to changes in ether physiological or work-load conditions.

## IN THE DRAWINGS

Figure 1 shows affinity chromatography separation of ammonium sulfate precipitated tissue culture supernatant protein on HPLC protein A preparative column.

Figure 2 shows the IgG chromatography on HPLC protein A analytical column.

Figure 3 shows the IgG chromatography on HPLC hydroxylapatite (HPHT).

Figure 4 shows the IgG chromatography on HPLC gel filtration column.

Figure 5 shows the antibody dilution curves assayed in ELISA.

Figure 6 shows the antigen concentration standard curves for human cardiac muscle myosin heavy chains (CHC) and human skeletal muscle myosin (SkM) in ELISA.

Figure 7 shows the tissue uptake of the labeled antibodies and fragments in PKD.

Figure 8 shows the tissue uptake of the labeled antibodies and fragments in a tissue to blood ratio.

Figure 9 shows the scintiscan of a dog infarcted heart.

## DETAILED DESCRIPTION OF THE INVENTION

The antibody of the present invention can be distinguished from the antibodies known in the art in that it has the characteristic of being able to recognize 2 isozymes of cardiac myosin. A further useful characteristic of the antibody of the present invention is that it is capable of recognizing both $\alpha$- and $\beta$-heavy chain of atrial and ventricular human myosin.

The antibody of the present invention was obtained from the polyethylene glycol fusion of non-secreting mouse myeloma (P3X63.Ag8.653) cells with spleen cells from a Balb/c mouse immunized with human ventricular myosin. Hybrid cells were selected in HAT medium, tested for antigenic specificity in ELISA, and subcloned by limiting dilution four times. The hybridoma ATCC HB9916 clone designated 3-48$G_5 C_7$ has been propagated in vitro, in a stationary phase monolayer tissue culture and is still stable after 37 passages. Antibody secreted to the tissue culture supernatant is concentrated and purified to homogeneity.

Both the primary cell seed and working cell bank are free of mycoplasma, viruses, bacteria and fungi. The cell line doubting tame is 18.2 hrs and specific antibody secretion rate 70 $\mu$g IgG/ml medium/24 hrs. The antibody has been characterized with respect to its immunoglobulin class, isoelectric point, affinity, molecular weight and immunologic specificity.

The method for preparation of this hybridoma and the properties of the antibody of the present invention are described in detail below.

### Obtaining hybridoma

### 1. Antigen preparation, isolation and purification of cardiac myosin heavy chains from human or canine left ventricle

All steps were performed at 4°C. Canine left ventricular muscle had beef stored in 50% glycerol in 0.06 M KCl, 0.02 M KP pH 6.5, 0.005 M $\beta$-ME, 0.005 M $MgCl_2$, 0.0002 M PMSF. The tissue was minced in a cold meat grinder previously washed with 0.2 M EDTA pH 7.0 and a resulting mince homogenized in 5 volumes of 0.05 M $KH_2PO_4$ pH 6.8, 0.001 M EDTA, 0.01 M NaPPi pH 7.5, 0.001 DTT, 0.03 M KCl, 0.01 M $MgCl_2$, 0.0001 M PMSF (solution 1) for 1 minute at 60 revolutions (27000 rpm) in Virtis 45 ® homogenizer.

The homogenate was centrifuged for 10 minutes at 8000 rpm (Sorvall GS-3 ®) and the pellet was washed once in 3 volumes of solution 1 and then once in 1% Triton in solution 1, followed by five washes in solution 1 to remove traces of Triton. Each time the pellet was resuspended by brief (3 seconds) homogenization at 27000 rpm. The final pellet was homogenized in 400 ml of 0.1 M $KH_2PO_4$, 0.3 M KCl, 0.01 M NaPPi, 0.001 M DTT, 0.001 M EDTA, 0.05 M $K_2HPO_4$,0.005 M $MgCl_2$, 0.00035 M ATP, 0.0002 M PMSF, 1 $\mu$g/ml pepstatin A, pH 7.5 and extraction continued for 15 minutes while stirring. Actomyosin containing supernatant obtained after centrifugation for 15 minutes at 8000 rpm (Sorvall GS-3 ®) was filtered through a cheese cloth and spun again for 20 minutes at 9000 rpm (Sorvall GS-3 ®). The actomyosin was precipitated from the supernatant with 9 volumes of $H_2O$, 0.001 M EDTA and collected by centrifugation at 9000 rpm (Sorvall GS-3 ®) for 20 minutes. The pellet was resuspended by homogenization in a glass Poter homogenizer in 87 ml of 0.05 M NaPPi, pH 7.5, 0.001 M DTT, 0.001 M EDTA, 0.005 M $MgCl_2$, 0.002 M ATP, and protein concentration determined by extinction coefficient ($E^{1\%}_{280nm}$ for myosin = 5.6). The myosin solution was made 2 mg/ml and contaminants (actin and tropomyosin) precipitated with 34% $(NH_4)_2SO_4$, 0.001 M EDTA, pH 6.8 for 30 minutes, stirring. The supernatant, containing myosin, was collected by centrifugation for 25 minutes at 10,000 rpm and precipitated again with 42% $(NH_4)_2SO_4$ for 30 minutes, stirring. This time the myosin containing precipitate was collected by centrifugation at 9000 rpm (Sorvall GS-3 ®) for 30 minutes. The pellet was resuspended in small volume of 0.05 M Tris/HCl pH 7.5, 0.5 M KCl, 0.001 M DTT and dialyzed against 200 volumes of the same solvent overnight at 4°C, with three buffer changes. The dialysate was then spun at 10,000 rpm (Sorvall SS-34 ®) for 10 minutes and myosin containing supernatant precipitated with 9 volumes of d.$H_2O$, 0.001 M EDTA. Nice precipitate formed. It was spun at 20,000 rpm (Sorvall SS-34 ®) for 15 minutes.

To isolate heavy chains of myosin, the pellet was resuspended in small volume of 8 M urea, 0.001 M DTT, 0.01 M EDTA, 0.05 M Tris, pH 7.5 and stirred for 2 hours at room temperature. Heavy chains were precipitated with 10 volumes of cold d.$H_2O$, 1 mM EDTA pH 7.5 overnight at 4°C. The pellet was collected by centrifugation at 20,000 rpm (Sorvall SS-34 ®) for 10 minutes, resuspended in small volume of 0.5 M KCl, 0.05 M Tris pH 7.5, 0.001 M EDTA and dialyzed against the same buffer for 24 hours with 3 changes of buffer. Thus produced myosin was mixed with equal volume of cold glycerol and stored aliquoted at -20°C. The final yield of pure myosin was 173.6 mg per 74 g wet tissue. Each purification step was monitored by mobility on SDS-PAGE. The isolated heavy chains of myosin were relatively pure.

## 2. Immunization protocol

Balb/c female mouse, 6 weeks old, was injected with human left ventricular muscle myosin in 0.6 M KCl, 0.05 M potassium phosphate pH 6.5 according to the presented immunization schedule.

| Date | Time Interval | Immunogen | Dose | Route |
|---|---|---|---|---|
| 19-06-85 | 0 | (1) in CFA* | 100 $\mu$g(1)/100 $\mu$l + 100 $\mu$l CFA | i.p.** |
| 27-06-85 | 1 week | (1) in IFA*** | 100 $\mu$g(1)/100 $\mu$l + 100 $\mu$l IFA | i.p. |
| 04-07-85 | 1 week | (1) in IFA | 100 $\mu$g(1)/100 $\mu$l + 100 $\mu$l IFA | i.p. |
| 22-07-85 | 18 days | (1) in IFA | 100 $\mu$g(1)/100 $\mu$l + 100 $\mu$l IFA | i.p. |
| 12-02-86 | 7 months | (2) in CFA | 50 $\mu$g(2)/ 50 $\mu$l + 50 $\mu$l CFA | i.p. |
| 27-02-86 | 2 weeks | (2) in IFA | 50 $\mu$g(2)/ 50 $\mu$l + 50 $\mu$l IFA | i.p. |
| 14-03-86 | 2 weeks | (2) only | 100 $\mu$g(2)/100 $\mu$l + buffer | i.p. |
| 22-03-86 | 1 week | (2) in saline | 100 $\mu$g(2)/100 $\mu$l + saline - final boosting | i.v.**** |

* CFA = complete Freund's adjuvant;

** i.p. = intraperitoneally;

*** IFA = incomplete Freund's adjuvant;

**** i.v. = intravenously

(1) Human left ventricular muscle myosin lot # 3 (35% $(NH_4)_2SO_4$ cut in 50% glycerol, 0.6 M KCl, 0.05 M K phosphate, pH 6.5). For injections 1 mg/ml protein stock was prepared in 0.6 M KCl, 0.05 M K phosphate pH 6.5. 1 ml of myosin at 1 mg/ml was emulsified with 1 ml of Freund's adjuvant (aqueous phase into oil).

(2) Human cardiac myosin heavy chains "Toronto" lot. Protein concentration (Lowry): 3 mg/ml. For injections 1 mg/ml stock solution was prepared by 1:3 dilution in 0.6 M KCl, 0.05 M $K_2HPO_4$ pH 6.7.

Hydridoma production and selection

As myeloma cells, cell lines originated from various animals such as mice, rats and humans can be used. The cell line preferably used was of the non-secretor type, more specifically P3X63.Ag8.653. Nucleated spleen cells of Balb/c female mice, immunized mouse cells. Spleen cells ($1.1 \times 10^8$) were combined with myeloma cells ($1.1 \times 10^7$) at a ratio of 10:1 and fused with 50% polyethylene glycol (PEG) M.W. 4000 (Merck) containing 5% DMSO (ATCC) at pH 7.4. PEG was added gradually: 1 ml over 1 minutes, left for 90 seconds at 37°C and then diluted with serum-free medium to 25 ml.

After washing, the fused cells were resuspended in HAT selective medium (IMDM, Pen/Strep, 2 mM L-Glutamine, 1 mM sodium pyruvate, 0.1 mM MEM non-essential amino acids, 0.025% insulin, 0.025% transferrin, 0.25 $\mu$g/ml sodium selenite, $5 \times 10^{-5}$ M 2-ME, HAT, 15% FBS) supplemented with ECGS (CR-endothelial cell growth supplement) (100 $\mu$g/ml) at $1 \times 10^6$ viable cells/ml and distributed into 6 24-well Costar plates 1 ml/well. Each well contained $1 \times 10^5$ normal mouse spleen cells in 1 ml of HAT medium to serve as a feeder layer.

Seven days later the clones were fed by replacing half of the volume with fresh HAT medium. On day 10, the plates were observed for hybrid growth and growing ones selected for screening in ELISA against heavy chains of human cardiac myosin and human skeletal myosin.

Clones whose supernatants were positive for both antigens were transferred to a 25 cm$^2$ flask and then to a 75 cm$^2$ T-flask. The hybridoma was subcloned by limiting dilution four times in IMDM supplemented as above.

As a result, a hybridoma ATCC HB9916 cell line producing an antibody having the ability to recognize cardiac myosin heavy chain $\alpha$ type and $\beta$ type and $\beta$-heavy chain of slow skeletal muscle was obtained. It was designated 3-48 $G_5C_7$.

Production of monoclonal antibody

The final subclone, ATCC HB9916, was grown in a stationary monolayer culture in IMDM 95%, fetal bovine serum 5%. The cultures were passaged once a week into a new flask and fed twice a weed by removing the spent medium by centrifugation and resuspending the cell at $0.25 \times 10^6$ $\ell$/ml in fresh medium. On average, 600 ml of tissue culture supernatant was collected per week.

1. ELISA for anti-myosin monoclonal antibody

Screening of tissue culture supernatants for anti-myosin monoclonal antibody production was done in enzyme-linked immunosorbent assay (ELISA). Microtitration plates (Dynatech Immulon I ®) were coated with 100 $\mu$l of human cardiac myosin heavy chains at 10 $\mu$g/ml in 0.05 M sodium carbonate-bicarbonate buffer pH 9.6, for overnight at 4°C. The unbound antigen was washed off 4 times with Tris buffered saline pH 7.4 (TS), 0.05% Tween 20 ® (TS-Tween) and the remaining binding sites saturated with 1% bovine serum albumin (250 $\mu$l/well) in TS for 1 hour at 37°C. After brief washing, the wells were incubated with 100 $\mu$l of tissue culture supernatants from growing hybrids for 1 hour at 37°C. The unbound antibody was washed off four times with TS-Tween® and horseradish peroxidase-conjugated anti-mouse IgG + IgM antiserum (BioCan Scientific #115-3568), diluted 1:5000 with TS-BSA, added (100 $\mu$l/well) for further 1 hour incubation at 37°C. The colorimetric reaction was developed upon the addition of 30% $H_2O_2$ (Fisher) at 1 $\mu$l/l ml in 0.1 M sodium citrate buffer pH 5.0 containing 0.1 % o-phenylenediamine dihydrochloride (Sigma), and the absorbance read at 450 nm.

2. Purification of immunoglobulin

Tissue culture supernatant of ATCC HB9916 hybridoma was five fold concentrated by ultrafiltration on PM 30 membrane and precipitated with 50% saturated ammonium sulfate, pH 6.8 for 60 minutes at 4°C, stirring. The precipitate was collected by centrifugation at 8000 rpm for 20 minutes and resuspended in PBS. It was dialyzed against PBS for two days with 4 buffer changes and then filtered through 0.45 $\mu$m membrane and further dialyzed against protein A binding buffer (1.45 M glycine, 3 M NaCl, pH 9.4) overnight at 4°C. 250 mg protein in 5 ml of binding buffer was injected into Bio-Rad ® protein A HPLC preparative column and IgG eluted with Bio-Rad ® protein A elution buffer pH 3.1 at 4 ml/min with the following gradient of Buffer B:

| Time (min) | % B |
|---|---|
| 0 | 0 |
| 28 | 0 |
| 28,1 | 100 |
| 55,1 | 100 |
| 55,2 | 0 |
| 60,2 | 0 |

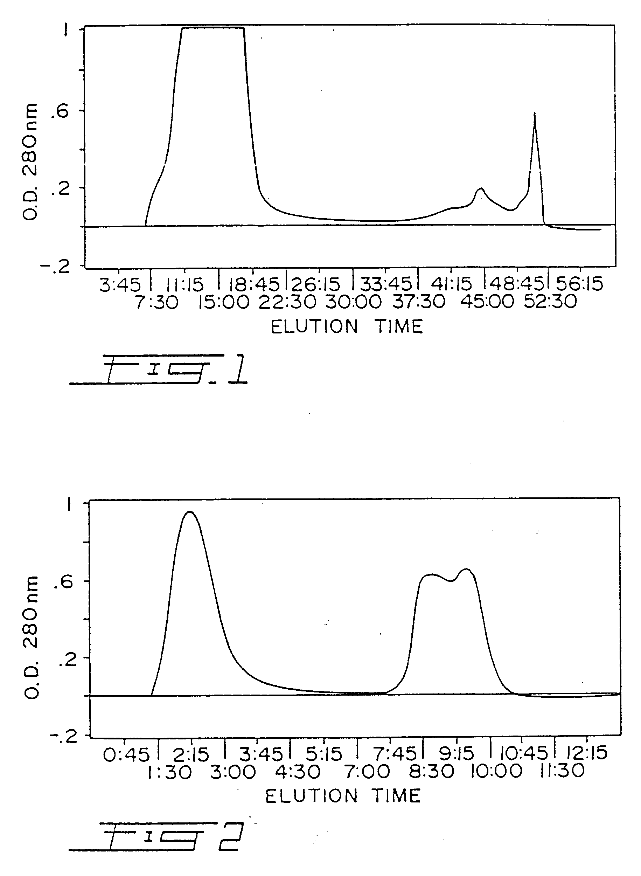

and 1 min (4 ml) fractions were collected (Figure 1).

The IgG containing fractions were pooled, neutralized with NaOH, concentrated by ultrafiltration on PM 30 ® membrane and dialyzed again against the protein A binding buffer. 33 mg IgG in 4.2 ml of binding buffer were then injected into Bio-Rad ® HPLC protein A analytical cartridge and eluted as before with Bio-Rad ® protein A elution buffer pH 3.1 at 4 ml/min with the following gradient of buffer B:

| Time (min) | % B |
|---|---|
| 0 | 0 |
| 4 | 0 |
| 4,1 | 100 |
| 9,1 | 100 |
| 9,2 | 0 |
| 12,2 | 0 |

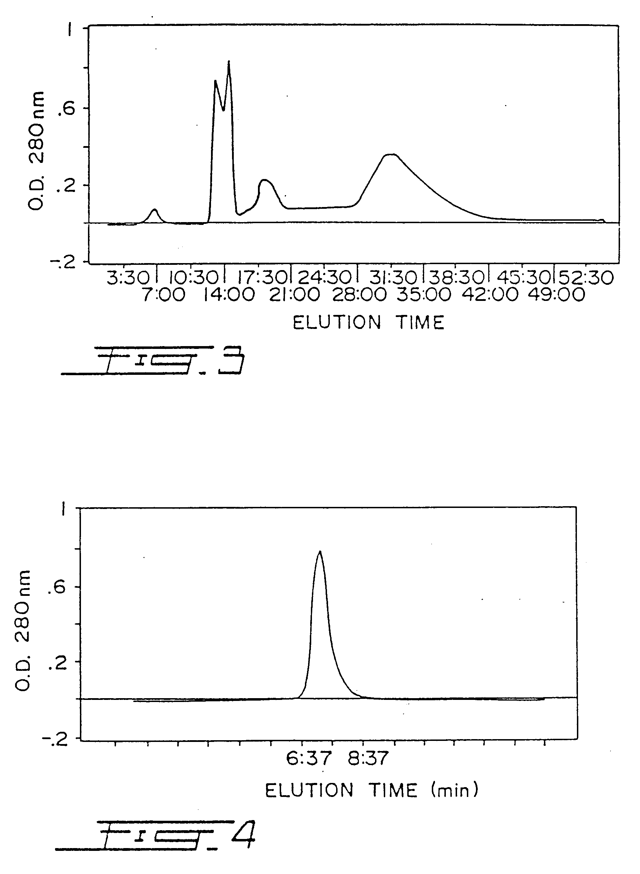

and 1 min (4 ml) fractions were collected (Figure 2).

The IgG containing fractions were pooled, neutralized with NaOH, concentrated by membrane ultrafiltration and dialyzed against 10 mM Na phosphate buffer, pH 6.8, 0.01 mM $CaCl_2$, 0.02% $NaN_3$ overnight at 4 °C (HPHT column buffer). 4.6 mg IgG in 2 ml of HPHT column buffer was injected into HPLC hydroxylapatide (HPHT) column and eluted at 1 ml/min with the following gradient of buffer B (350 mM) Na phosphate buffer pH 6.8, 0.01 mM $CaCl_2$:

| Time (min) | % B |
|---|---|
| 0 | 0 |
| 7 | 18 |
| 14 | 19 |
| 15 | 22 |
| 22 | 23 |
| 23 | 27 |
| 30 | 27 |
| 35 | 30 |
| 50 | 30 |
| 53 | 40 |
| 56 | 40 |
| 0 | 61 |

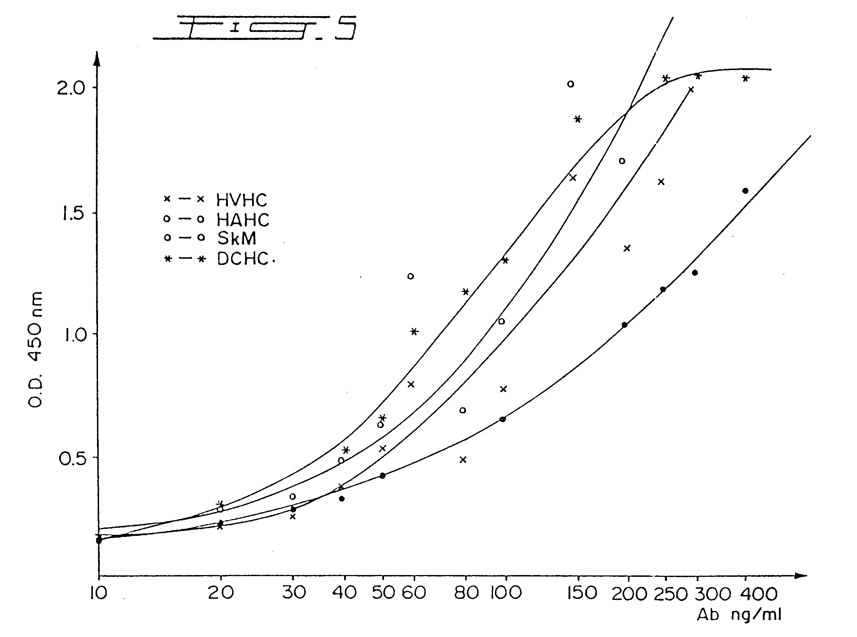

and 1 min (1 ml) fractions were collected (Figure 3).

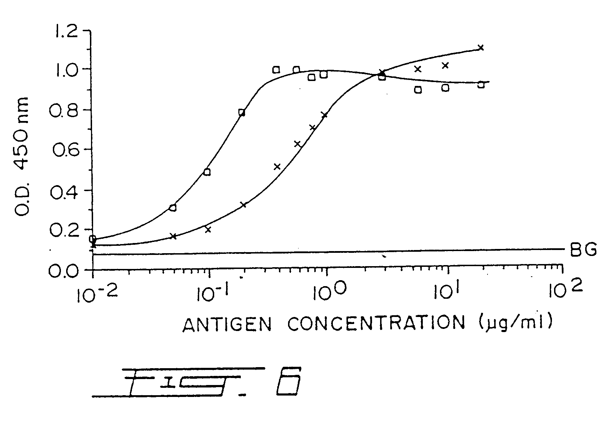

The last peak, containing IgG was pooled, concentrated by membrane ultrafiltration and dialyzed overnight at 4 °C against 0.05 M $Na_2SO_4$, 0.02 M $NaH_2PO_4$, pH 6.8 buffer. 1.0 mg IgG in 1.0 ml of the above buffer was injected into HPLC TSK 250 gel permeation column (GFC) and chromatographed for 11 minutes at 1.0 ml/min (Figure 4). The IgG containing fractions were then pooled, concentrated by membrane ultrafiltration and dialyzed against PBS. For storage, protein concentration was adjusted to 5 mg/ml by Lowry assay and IgG aliquoted at 5 mg per vial for lyophilization. Lyophilized IgG was stored at -20 °C.

7

### F(ab')₂ fragment isolation from IgG₁

Limited proteolysis of IgG with the enzyme pepsin has been widely used for the preparation of F(ab')₂ fragments. Because there is variation among individual monoclonal antibodies of each subclass with respect to the rate of digestion, such parameters as optimal pH and incubation period had to be determined experimentally to obtain maximum yields of F(ab')₂ fragments (and little intact IgG).

The production of F(ab')₂ from IgG₁ was studied as a function of: pH in the range of 3.5 - 4.2, time in the range of $1\frac{1}{2}$ - 24 hours and enzyme-antibody ratio of 1:10 - 1:100. The digestions were performed at 37°C at 1 mg IgG/ml. The digestion was stopped by adding 1/40 volume of 1 M Tris HCl and raising the pH to 8.0. The course of digestion was monitored by SDS-PAGE under non-reduced conditions and retention of antibody activity by serial dilutions in ELISA.

The rate of digestion increased as the pH was decreased; at pH 4.2 the reaction required 24 hours for completion and at pH 3.5, cleavage was complete after 8 hours. The optimal conditions for preparation of F-(ab')₂ fragments were found to be 4 hour digestion at pH 3.9 and pepsin-IgG ratio of 1:50. Under those conditions F(ab')₂ fragments were obtained in 60% yield.

Traces of undigested IgG and Fc fragment were removed by a passage through the HPLC protein-A column. The purified F(ab')₂ fragments of 3-48 IgG still retained their reactivity with cardiac myosin when assayed in ELISA and on cryostat sections of ventricular muscle by indirect immunofluorescence.

### Characterization of mouse monoclonal anti-myosin antibody

#### 1. Immunoglobin class determination

The immunoglobulin isotype was determined by double immunodiffusion in 1% agarose against monospecific, commercially available anti-mouse immunoglobulin class and subclass specific antisera. The immunoglobulin class of the antibody is IgG₁/k.

#### 2. Determination of affinity constant of IgG

The equilibrium constant for hybridoma ATCC HB9916 and dog heavy chains (DHC) of cardiac myosin interaction by solid-phase enzyme immunoassay for antigen was evaluated. The assay (by means of ELISA) is based on competition for the antibody between free antigen and antigen adsorbed to polystyrene (PJ Hogg et al., Mol. Immunol. 1987; 24:797-801). The assay follows the general procedure in which ELISA plates were first coated with DHC at 10 μg/ml in 100 μl of coating buffer and incubated overnight at 4°C. Next day the excess of antigen was removed by washing each well 4 times with TS-Tween ®. Reaction mixtures (100 μl/well) containing 100 ng/ml (0.65 nM) 3-48 G₅C₇ lot # 2 IgG and DHC lot # 15 (0-15 μM) in 20 mM Tris/HCl, 0.5 M NaCl, pH 7.4 were placed in half of the coated wells, in triplicates. To the remaining wells, which were used for analysis of the interaction between IgG and immobilized DHC in the absence of soluble antigen was added the monoclonal 3-48 IgG (100 μl; 0-65 μM) in 20 mM Tris/HCl pH 7.4, 0.5 M NaCl. After incubation at 37°C for 1 hour, the plates were washed 4 times with TS-Tween prior to the addition of peroxidase-labelled polyclonal antibody directed to IgG + IgM (H + L) (Jackson Lab. # 5839) for a further 1 hour at 37°C. Excess second antibody was then removed by washing, peroxidase substrate added and absorbance measured at 450 nm. Affinity constant $K_{AS}$ was measured from the formula:

$$\frac{K_{AS}}{(1 \div 2\, K_{AS}\, m_A)} = \text{slope of the curve representing binding of antibody in the presence of free antigen.}$$

Where $K_{AS}$ is slope of the curve described by:

$$\frac{A_{450\ nm}}{m_A} \quad \frac{\text{(Ab bound to matrix in the absence of free antigen)}}{\text{(concentration of added antibody in uM)}}$$

| Affinity constant for: | | |
| --- | --- | --- |
| Antigen | IgG | F(ab')$_2$ |
| Human ventricular myosin heavy chains | $3.33 \times 10^8 \, M^{-1}$ | ND |
| Human skeletal muscle myosin | $1.06 \times 10^8 \, M^{-1}$ | ND |
| Canine cardiac myosin heavy chains | $2.06 \times 10^8 \, M^{-1}$ | $1.21 \times 10^8 \, M^{-1}$ |
| Human atrial myosin heavy chains | $1.48 \times 10^8 \, M^{-1}$ | ND |

3. Determination of the isoelectric point of 3-48 IgG$_1$

The isoelectric point (pI) of anti-myosin antibody was determined by isoelectrofocusing (IEF) in Pharmacia's ® Phast Gel IEF 3-9 medium in a pre-cast homogenous polyacrylamide gel containing Pharmalyte ® carrier ampholytes in the 3 to 9 pH range. When the immunoglobulin sample was applied at either cathode or anode end, its pI was between 6.85 and 6.55, while when applied in the middle of the gel, it was closer to 6.55 (6.85-5.85).

4. Immunospecificity and cross-reactivity of the antibody

4.1. ELISA

The immunologic potency of the antibody of the present invention towards its target antigen, ventricular myosin heavy chains, and cross-reacting antigen, skeletal muscle myosin heavy chains, was quantitated by antibody titration and measurement of an affinity constant in ELISA.

Briefly, antigens were immobilized on polystyrene microtiter plates (Immunlon 1, Dynatech Labs) at a concentration of 10 $\mu$g/ml in 0.1 M sodium carbonate/bicarbonate buffer, pH 9.6 overnight at 4°C. Before use, the plates were washed and incubated for 1 hour at 37°C with 1% low-fat powder milk in Tris-saline (TS). Antibody dilutions in TS containing 1% milk were incubated at 37°C for 1 hour. After washes, the plates were incubated with peroxidase-conjugated anti-mouse IgG ( H + L) (Jacksons Lab) diluted in TS-milk for 1 hour at 37°C, then washed, and the enzyme activity revealed with o-phenylenediamine (Sigma) at 1 mg/ml in 0.1 M sodium citrate-citric acid buffer, pH 5.0, containing 0.03% H$_2$O$_2$. The colour was read after 30 minutes in an automatic reader using interference filter of 450 nm.

Typical sigmoid serial dilution ELISA curves were obtained for all antigens tested (human cardiac and skeletal myosin, canine cardiac myosin, porcine, bovine, rabbit and chicken skeletal muscle myosins, chicken gizzard myosin). As it was found from the antibody concentrations required to reach 50% binding capacity, the IgG of the present invention has the highest affinity for human skeletal myosin, and then for human cardiac, bovine skeletal, canine cardiac, porcine skeletal and the lowest for rabbit skeletal muscle myosin. There was no reactivity towards chicken gizzard nor chicken skeletal muscle myosin.

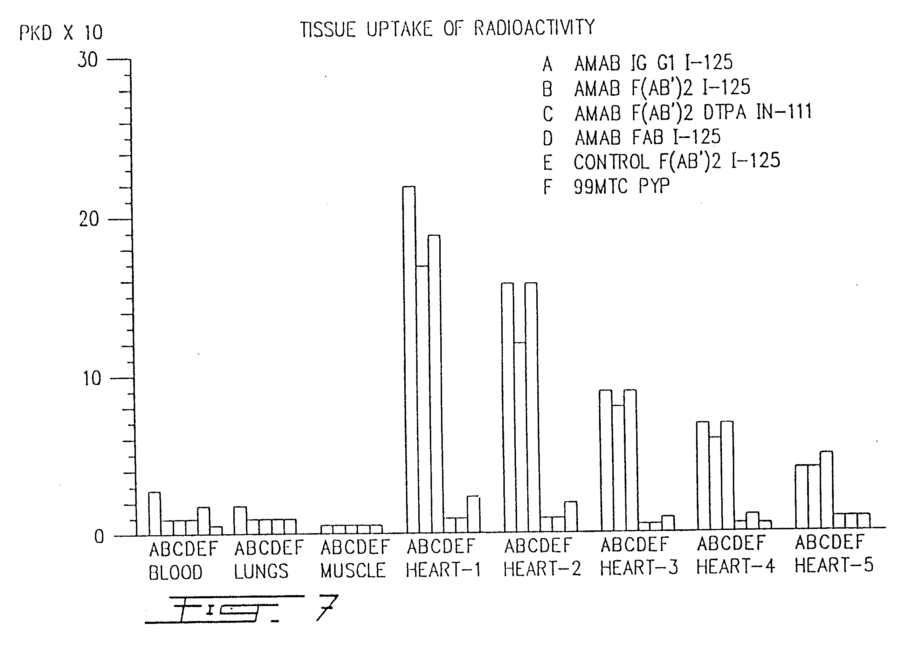

When tested against sheep or human atrial myosin and light chain 1 (HVLC$_1$) and light chain 2 (HVLC$_2$) of human ventricular myosin in ELISA, the antibody was found to react with atrial heavy chains but not with light ventricular chains (Figure 5). The antibody dilution curves for 3-48 IgG were measured against human ventricular myosin heavy chains (HVHC), human atrial myosin heavy chains (HAHC), human skeletal myosin heavy chains (SkM), and canine ventricular myosin heavy chains (DCHC) in ELISA. Thus, immunoglobulin reacts with $\alpha$- and $\beta$-heavy chains of myosin, since human ventricular myosin is composed primarily of the $\beta$-type of heavy chains that share antigenic determinants with slow skeletal muscle myosin heavy chains, and human atrium of the $\alpha$-type primarily.

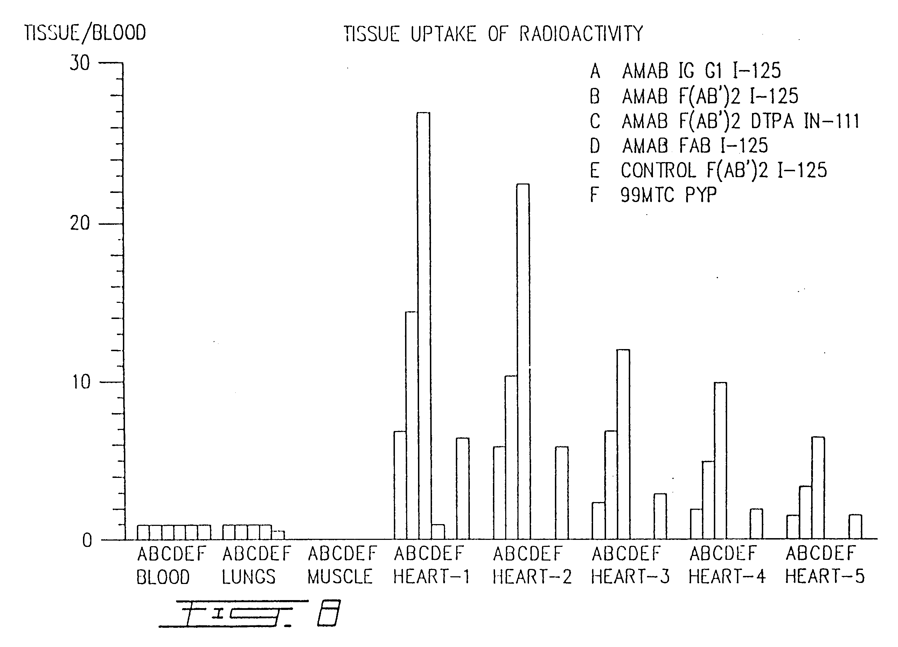

The potency of the McAb was also on occasion quantitated by constructing antigen concentration curves for which microtitration plates were coated with ascending concentrations of antigen (CHC, SkM or DHC) and subsequently incubated with the IgG at the concentration equivalent to 50% of its maximum binding to the corresponding antigen, e.g. 40 ng/ml for SkM, 110 ng/ml for DHC, and 60 ng/ml for CHC. A typical antigen concentration curve is presented in Figure 6.

Microtitration plates were coated with rising concentrations of either CHC (x-x) or SkM (□ - □) and incubated with the IgG$_1$ concentrations representing 50% of maximum binding (60 ng/ml for CHC and 40 ng/ml for SkM). The bound antibody was detected by incubation with peroxidase labelled anti-mouse IgG.

In case of SkM, antibody saturation point is achieved with 0.4 ug/ml of SkM while for CHC saturation point is reached with about 4 ug/ml of CHC.

4.2 Immunohistochemical staining of tissue sections with the antibody of the present invention.

Cryostat cuts of either human or animal fresh autopsy tissues were prepared on gelatinized slides and incubated with the present antibody at various dilutions in PBS for 1 hour at room temperature. The slides were washed in PBS and incubated for an additional one hour with either fluorescein or peroxidase (after quenching of endogenous peroxidase with 0.3% $H_2O_2$ in methanol) conjugated F(ab')$_2$ fragments of goat anti-mouse IgG. After washing as above, the slides were observed under either the fluorescence or light microscope.

The antibody stained only striated muscle fibers in both atria and ventricles of human and animal hearts, as well as skeletal muscle fibers. On longitudinal cryosections, the staining was restricted to the A bands of a myofibril, indicating that the antibody recognizes only myosin (S. Lowey, Med. Sci. Sport. Excer. 18, 284-291, 1986). No staining was observed with other tissue antigens.

When incubated with human or rat cardiac myocytes in culture, the 3-48 antibody stained only cytoplasm and not nuclei or fibroblasts.

Since human atrial muscle contains also $\beta$ chain, as well as human ventricular muscle contains also $\alpha$ chain, neither of those muscles can be considered a source of pure $\alpha$ or $\beta$ heavy chains. To prove beyond doubt that the antibody 3-48 recognizes both $\alpha$ and $\beta$ chains, cryocuts of rat hearts at various developmental or physiological stages were prepared.

It has been shown (C. A. Dechesne et al., J. Cell Biology, 1987; 105: 3031-3037) that ventricles of:

- 20 to 25 day old Wistar male rats are composed of only homodimers,
- 6 month old Wistar male rats contain both homo and heterodimers of $\alpha\alpha$, $\alpha\beta$ and $\beta\beta$ heavy chains,
- 7 month old Wistar male rats thyroidectomized at 3 months of age contain only $\beta\beta$ homodimers.

If 3-48 antibody recognizes only $\alpha$ chain it will not stain thyroidectomized rat ventricles while if it recognizes exclusively $\beta$ chain it will not stain 20-25 day old rat ventricles.

The antibody was found to stain (by indirect immunofluorescence) all three types of ventricular tissue sections equally well, indicating that 3-48 antibody has been raised to common epitopes on $\alpha$ and $\beta$ myosin heavy chains.

4.3 Immunodetection on Western blots.

Sodium dodecyl sulfate-polyacrylamide gel electrophoresis (SDS-PAGE ®) was performed on 8% acrylamidebisacrylamide running gel with 5% stacking gel in a Tris-glycine discontinuous buffer system of Laemmli (Laemmli, Nature, 227, 680-685, 1970) in a vertical slab gel apparatus. The electrophoretically resolved proteins were transferred at 35V for 4 hours in 25 mM Tris, 192 mM glycine, 20% methanol, pH 8.3 to nitrocellulose sheets according to the method of Towbin (Towbin, H. et al.; Proc. Natl. Acad. Sci., 76, 4350-4354, 1979).

The remaining binding sites on nitrocellulose were quenched by overnight incubation at 4°C in 5% BSA in TS. The blots were then incubated for 3 hr at room temperature, while shaking, with 3-48 antibody diluted in TS-1% BSA and then for 2 hr with peroxidase conjugated anti-mouse IgG. The enzymatic reaction was carried out by over-laying the strips with 0.03% $H_2O_2$ in diaminobenzidine at 1 mg/ml TS. Each of the steps was followed by extensive washing with TS-Tween 20®. Proteins electrotransferred to nitrocellulose were visualized by staining with 0.1% amido black in 45% methanol, 7% acetic acid and destained in the same solvent.

When atrial, ventricular and skeletal muscle myosin subunits were separated by SDS-PAGE® and transferred to nitrocellulose, the 3-48 antibody reacted specifically with only the 200 kd band, corresponding to the myosin heavy chain.

In summary, the monoclonal antibody is myosin $\alpha$- and $\beta$-heavy chain specific. On Western blots and in ELISA it reacts with purified heavy chains of atrial and ventricular human myosin. It binds to A-band of a thick filament of cardiac and skeletal muscle myosin as determined by indirect immunofluorescence on cryostat tissue sections, and indirect immunoperoxidase staining of Carnoy fixed tissues. It stains human and canine myocytes in culture as determined by indirect immunofluorescence.

It cross-reacts strongly with heavy chain of slow human skeletal muscle myosin but it neither binds with human myosin light chains nor smooth muscle myosin. It cross-reacts with canine, porcine, bovine, rabbit and rat $\beta$ heavy chain of myosin, and sheep $\alpha$ chain of myosin. Other animal species were not tested. The antibody does not cross-react with any human tissue antigens or blood cells.

5. Karyotypic analysis

The test article, Hybridoma Cells 3-48 $G_5C_7$, was characterized by analysis of the mobility of glucose 6-phosphate dehydrogenase ($G_6PD$), nucleoside phosphorylase (NP), lactate dehydrogenase (LD), and malate dehydrogenase (MD) enzymes and by Giemsa banded chromosome analysis.

A. Isoenzyme Analysis of Cell Line ATCC HB9916:

Electrophoretic mobilities of enzymes $G_6PD$, NP, LD, and MD present in an extract prepared from 3-48 $G_5C_7$ cells were comparable to those of a mouse cell preparation. No extra bands were found that would suggest the presence of another cell species.

B. Cytogenetic Analysis of ATCC HB9916:

I. Chromosome count per 100 Metaphases:

The distribution of chromosome numbers found in the 100 metaphases analyzed for cell line 3-48 $G_5C_7$ is shown in Table 1. The chromosome count range from 47 to 93 chromosomes per metaphase.

TABLE I

| DISTRIBUTION OF CHROMOSOME NUMBERS IN THE 100 METAPHASES ANALYZED FOR CELL LINE ATCC HB9916 | |
|---|---|
| Number of Chromosomes | Number of Metaphases |
| 47 | 1 |
| 67 | 2 |
| 70 | 1 |
| 71 | 1 |
| 72 | 2 |
| 73 | 6 |
| 74 | 2 |
| 75 | 10 |
| 76 | 3 |
| 77 | 11 |
| 78 | 8 |
| 79 | 5 |
| 80 | 17 |
| 81 | 3 |
| 82 | 7 |
| 81 | 3 |
| 82 | 7 |
| 83 | 10 |
| 84 | 1 |
| 85 | 3 |
| 86 | 3 |
| 87 | 2 |
| 90 | 1 |
| 93 | 1 |

2. Chromosome Aberration Data:

The chromosome aberration data for the 50 metaphases examined is summarized in Table 2.

## TABLE II

## CHROMOSOME ABERRATION DATA FOR CELL LINE ATCC HB9916

### TYPE OF ABERRATION

| Chromatide | | Chromosome | | Severely | No.Cells | Total No. |
|---|---|---|---|---|---|---|
| Deletion | Interchg. | Deletion | Interchg. | Damgd.Cell | Aberrant | Aberration |
| 6 | 0 | 1 | 0 | 0 | 7 | 7 |

# Cells analysed = 50
# Aberrations = 7
# Cells with Aberrations = 7
% Cells with Aberrations = 14
# Aberrations/Cell = 0.14

Seven chromosome aberrations were found in the 50 cells analyzed with 14 percent cells aberrant.

3. Giemsa Banded Chromosome Analysis/Karyotypes:

Five karyotypes were prepared. Cytogenetic examination shows that the cell line is of "mouse" origin.

Cell line ATCC HB9916 showed a wide and highly variable distribution of normal chromosomes, chromosomal rearrangements, and markers. Due to such variability in the karyotype, all unidentifiable chromosomes as well as all chromosomes containing rearrangements were collectively classified as markers. Markers ranged in numbers from 58-64 in the 5 cells karyotyped.

## TABLE III

## DISTRIBUTION OF NORMAL CHROMOSOMES & NUMBER OF MARKER CHROMOSOMES IN THE 5 KARYOTYPES ANALYSED FOR CELL LINE ATCC HB9916

No. of Copies of Normal Chromosomes and Unidentified Marker Chromosomes (M)

| Karyotype No. | 1 | 2 | 3 | 4 | 5 | 6 | 7 | 8 | 9 | 10 | 11 | 12 | 13 | 14 | 15 | 16 | 17 | 18 | 19 | X | Y | M |
|---|---|---|---|---|---|---|---|---|---|---|---|---|---|---|---|---|---|---|---|---|---|---|
| 1 | 0 | 2 | 2 | 0 | 0 | 2 | 1 | 0 | 1 | 2 | 2 | 1 | 2 | 2 | 0 | 0 | 1 | 1 | 0 | 1 | 0 | 61 |
| 2 | 2 | 1 | 2 | 0 | 1 | 0 | 1 | 0 | 2 | 1 | 2 | 0 | 0 | 2 | 0 | 1 | 0 | 0 | 3 | 1 | 0 | 64 |
| 3 | 2 | 2 | 1 | 0 | 1 | 1 | 1 | 0 | 2 | 2 | 2 | 0 | 1 | 1 | 0 | 0 | 0 | 0 | 1 | 0 | 0 | 53 |
| 4 | 3 | 4 | 0 | 0 | 1 | 0 | 1 | 1 | 2 | 1 | 2 | 0 | 2 | 1 | 2 | 0 | 2 | 0 | 1 | 0 | 0 | 60 |
| 5 | 2 | 3 | 1 | 0 | 2 | 0 | 0 | 0 | 2 | 3 | 3 | 0 | 1 | 2 | 1 | 0 | 1 | 0 | 0 | 0 | 0 | 62 |

No attempt was made to describe the origin of the numerous chromosomal rearrangements due to the degree of variability of the karyotype.

Normal chromosomes were found as absent, present, or in multiple copies depending on the cell analyzed. Chromosome #4 and the Y chromosome were absent in all karyotypes.

## 6. Molecular weight of IgG

| acc. to SDS-PAGE | | |
|---|---|---|
| under ME reduced conditions: | 153.8 kd | 49.7 kd heavy chain<br>27.2 kd light chain |
| no ME reduction:<br>acc. to gel filtration: | 253.7 kd<br>170 kd | |

Molecular weight of F(ab')$_2$ fragment:

| acc. to SDS-PAGE | | |
|---|---|---|
| under ME reduced conditions: | 120 kd | 30.5 kd heavy chain<br>29.5 kd light chain |
| no ME reduction:<br>acc. to gel filtration: | 127 kd<br>80 kd | |

Molecular weight of Fab' fragment

| acc. to SDS-PAGE | | |
|---|---|---|
| under ME reduced conditions: | 46.5 kd | 30.5 kd heavy chain<br>29.5 kd light chain |
| no ME reduction:<br>acc. to gel filtration: | 51.5 kd<br>39.0 kd | |

## Tissue staining with the antibody of the present invention

### 1. Radioimmunocalization of labelled antibody in animal model of myocardial infarction

$^{125}$I-labelled antibody was tested in a rat myocardial infarct (MI) model. Myocardial ischemic injury was induced with isoproterenol and resulted in increased cardiac uptake of TC-99m-PYP which is selectively retained by necrotic myocardium.

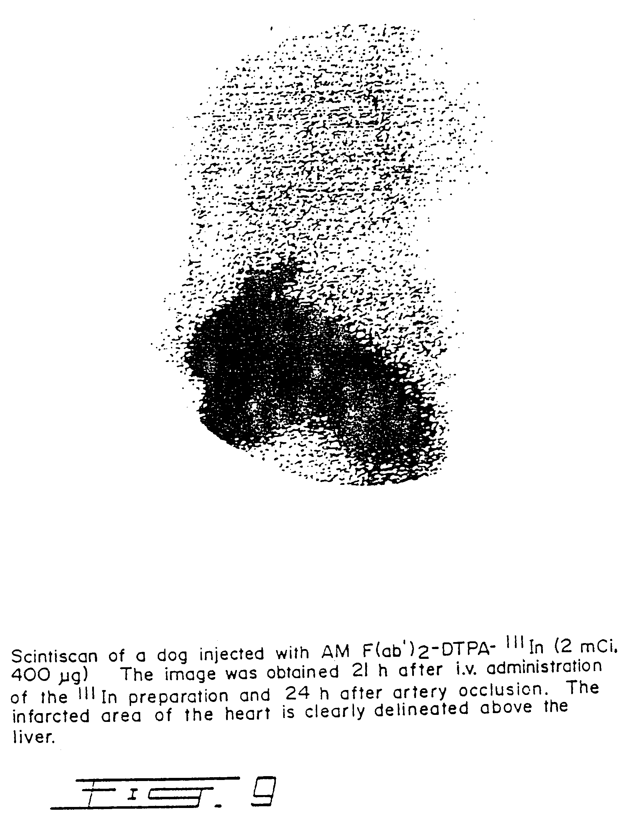

The IgG$_1$, and its Fab' and F(ab')$_2$ fragments as well as F(ab')$_2$ fragments from normal mouse IgG were $^{125}$I-labelled by Iodogen method. Rats were injected twice with isoproterenol and four hours after the second injection they were innoculated i.v. with labelled antibody or $^{99m}$Tc pyrophosphate. The animals were sacrificed after 20 hours and their hearts excised and blood, lung and skeletal muscle samples were collected. The data (Tables IV-V and Figures 7 and 8) clearly indicate that $^{125}$I-labelled antibody F(ab')$_2$ fragments exhibit the best retention in the infarcted rat heart, with high tissue to blood ratio, twice higher than the one for $^{99m}$Tc PYP.

TABLE IV

| TISSUE UPTAKE IN PKD X 1000a (S.D.)b | | | | | | | | |
|---|---|---|---|---|---|---|---|---|
| No. COMPOUNDS | BLOOD | LUNG | MUS. | T1 | T2 | T3 | T4 | T5 |
| 1 AMab Ig G1-(I-125) | 287 (28) | 208 (121) | 37 (22) | 2221 (578) | 1599 (308) | 926 (247) | 675 (241) | 420 (150) |
| 2 AMab F(ab')2 (I-125) | 121 (29) | 97 (40) | 22 (6) | 1698 (823) | 1205 (437) | 749 (421) | 606 (313) | 398 (173) |
| 3 AMab F(ab')2 DTPA (In-111) | 68 (18) | 76 (3) | 26 (1) | 1868 (585) | 1583 (473) | 871 (325) | 736 (335) | 532 (235) |
| 4 AMab Fab-(I125) | 95 (17) | 67 (28) | 16 (4) | 111 (33) | 82 (31) | 57 (20) | 51 (18) | 58 (16) |
| 5 Control F(ab')2 (I-125) | 189 (32) | 111 (17) | 16 (3) | 93 (60) | 85 (40) | 79 (28) | 78 (26) | 82 (30) |
| 6 99 mTc PYP | 35 (12) | nil nil | nil nil | 225 (37) | 182 (48) | 113 (61) | 64 (33) | 62 (18) |

a. PKD, percent kg dose, $((\mu Ci/g$ organ$)/(\mu Ci$(dose / kg body wt$)))$x 100.
b. Standard deviation.

TABLE V

| TISSUE TO BLOOD RATIO (S.D.)a | | | | | | | | |
|---|---|---|---|---|---|---|---|---|
| No. COMPOUNDS | BLOOD | LUNG | MUS. | T1 | T2 | T3 | T4 | T5 |
| 1 AMab Ig G1-(I-125) | 1 (0) | 0.71 (0.36) | 0.13 (0.06) | 7.75 (2.01) | 5.55 (0.80) | 3.19 (0.52) | 2.31 (0.56) | 1.46 (0.46) |
| 2 AMab F(ab')2 (I-125) | 1 (0) | 0.78 (0.13) | 0.18 (0.02) | 15.35 (8.94) | 10.69 (5.12) | 6.74 (4.80) | 5.38 (3.63) | 3.38 (1.68) |
| 3 AMab F(ab')2 DTPA (In-111) | 1 (0) | 1.18 (0.30) | 0.41 (0.12) | 27.45 (4.03) | 23.36 (3.39) | 12.75 (2.33) | 10.73 (3.18) | 7.62 (1.77) |
| 4 AMab Fab-(I125) | 1 (0) | 0.69 (0.15) | 0.17 (0.01) | 1.15 (0.17) | 0.84 (0.15) | 0.59 (0.10) | 0.52 (0.08) | 0.6 (0.09) |
| 5 ControlF(ab')2 (I-125) | 1 (0) | 0.59 (0.02) | 0.09 (0.01) | 0.47 (0.22) | 0.44 (0.13) | 0.41 (0.08) | 0.41 (0.07) | 0.43 (0.09) |
| 6 99mTc PYP | 1 (0) | nil nil | nil nil | 6.75 (1.49) | 5.56 (2.30) | 3.48 (2.30) | 2.01 (1.27) | 1.85 (0.74) |

a. Standard deviation.

There is no accumulation of antibody or its fragments in the normal heart or skeletal muscle nor is there any binding of the non-immune mouse IgG in the infarcted area.

2. $^{111}$In-labelled procedure.

2.1 Rat model. Protein labelling complex was obtained by reacting the antibody (135 $\mu$g) with an excess of DTPA anhydride at 4°C for 5 hours, with occasional stirring, whereafter excess DTPA was removed via centrifugation of the reaction mixture over a packed G-50 Sephadex ® column (0.8 ml in a 1 ml seringe) in 0.9% saline.

14

For labelling, the commercially obtained $^{111}$In chloride was converted to the acetate by mixing $^{111}$InCl with a 0.5 M sodium acetate solution (pH 4). The DTPA-protein complex was added to the $^{111}$In acetate solution (200 uCi), and left at room temperature for 1 hour. The mixture was loaded on a Sephadex G-50 ® column (1 x 20 cm) and eluted with a 0.05 M Hepes, 0.9% saline buffer. The labelled antibody (labelling efficiency 25-40%) was collected in the 6 to 8 ml fraction, and was detected by its UV absorption at 280 nm. The labelled antibody was filtered over a 0.22 um millipore filter prior to injection in the animals. Retention data of the labelled products in the various tissue samples are summarized in Table IV, tissue to blood ratios are presented in Table V. Data are also presented as bar plots to facilitate comparison of the various products (Figures 7 and 8).

In this animal model, the degree of heart infarction is the highest in the tip slice $T_1$, with gradual diminishing of the infarction from $T_1$ -$T_5$ and with the cranial slice $T_5$ almost appearing as unaffected healthy tissue. Accordingly, $T_5$ may be taken as an internal control of the healthy heart muscle.

It may be seen from the data (Tables IV and V) that the $^{125}$I and $^{111}$In-labelled AMab F(ab')$_2$ fragment exhibits the best retention in the infarcted rat heart, with a 2 to 4 x higher tissue to blood ratio as compared to $^{99m}$Tc PYP. The $^{111}$In DTPA-AMab F(ab')$_2$ probably due to a lower blood retention and a somewhat better tissue uptake reached much higher tissue to blood ratio than its $^{125}$I-labelled counterpart.

Since the $^{111}$In labelling efficiency of 25-40% obtained in the preliminary experiment is unsatisfactory for human use due to excess of free isotope, the conditions of labelling had to be optimized and standardized.

Sterile, apyrogenic and heavy metal ions free antibody F(ab')$_2$ fragments of IgG were covalently linked with DTPA by the cyclic anhydride method at the molar ratio of 1:100. An aliquot of 0.5 mg DTPA - antibody F(ab')$_2$ was dissolved in 100 $\mu$l PBS, and 1.8 ml of 0.1 M citrate (pH 5.0) was added. To this mixture, 2 mCi of $^{111}$In was added, and incubated at room temperature for 10-15 minutes. A small aliquot (0.01 ml) was used to determine labelling efficiency by ascending thin layer chromatography on cellulose acetate sheets developed in 0.1 M citrate, pH 5.0. The ratio of radioactive counts at the origin to that of the solvent front was used to compute labelling efficiency. Samples with 85% incorporation of $^{111}$In determined after 15-30 minutes of incubation at room temperature were considered usable. On two occasions so far, labelling efficiency of 90 and 95% have been achieved.

The immunologic specificity of DTPA chelated antibody fragments was tested in an inhibition ELISA and by determining an affinity constant in ELISA. The chelated fragments were still reactive towards its antigen but their potency was reduced by 20%.

When radiolabelled with $^{125}$I or $^{111}$In, and injected i.v. into rats with isoproprenolol induced myocardial infarction four hours after infarct induction, the antibody localizes only in the cardiac necrotic tissue. The label accumulation was proportional to infarct size and location was superior for F(ab')$_2$ fragments of immunoglobulin, when compared to intact molecule or Fab fragment. The maximum heart to blood ratio was 28:1.

### 2.2 MI dog model

The experimental infarctus was obtained by selective obstruction of a coronary artery with an artificially induced blood clot. The procedure is preferred over ligation since it does not require open chest surgery and allows for rapid recovery of the animal.

Adult female mongrel dogs (50-70 lb) were used. Animals were pretreated with Rompun prior to the procedure. A venous infusion line was placed in the cephalic vein and the dog was infused with penthobarbital. A guide catheter was introduced in the femoral artery and brought under fluoroscopic control into the acrtic root. The coronary anatomy was deleniated angiographically and the guide catheter was introduced into the guide up to the coronary artery and microfibrillar collagen preparation (Avitene Alcon Lab Inc., Forth Worth Texas, USA) was injected to block the artery. An injection of contrast agent was used to confirm the obstruction. The level of cardiac enzyme CPK in the blood was monitored before and at 5 and 20 h post infarctus. The animals were sacrificed 5 days post occlusion by bleeding under deep penthobarbital anesthesia. The heart was removed, washed and sent for pathologic examination and tetrazolium blue coloration.

The doses and intervals between artery occlusion and radiopharmaceutical administration were: 5 h for the Fab- and F(ab')$_2$- DTPA-$^{111}$In (2 mCi, 400$\mu$g) and 48 h for $^{99m}$Tc-PYP (10 mCi). The intervals between radiopharmaceutical injection and imaging were: 5,20 and 40 h for the $^{111}$In preparations and 3 h for the $^{99m}$Tc-PYP.

One dog was sacrificed 20 h post-injection of F(ab')$_2$-DTPA- $^{111}$In and 3 h post-injection of $^{99m}$Tc-PYP. The heart was removed, cut in 1 cm thick slices perpendicular to the ventricular axix. The heart slices were imaged using a gamma camera with one window set on the 140 keV peak of $^{99m}$Tc and the other on the 247 keVpeak of $^{111}$In. A small window opening (10%) was used to avoid spilling from the 178 keV gamma of $^{111}$In into the $^{99m}$Tc window imaging. Preliminary scintigraphic studies in the dog gave good images of the necrotic myocardium 20 h post-injection with either $^{111}$In-labeled Fab or F(ab')$_2$ (Figure 9). Earlier scans (5 h) gave a too high blood-background while later scans (48 h) did not improve the image obtained at 20-24 h. The dog heart images suggest that the AMab derived preparations visualize necrotic zones only, in contrast with conventional $^{99m}$Tc-PYP preparations which visualize both ischemic and necrotic tissues. Another advantage of the antibody preparation over $^{99m}$Tc-PYP is the absence of activity in the bones. More extensive scintigraphic studies with dogs will be required in order to assess possible differences in liver and infarction uptake between the Fab and F(ab')$_2$ preparations as well as to predict the extent of their usefulness in a clinical setting.

The deposit of the hybridoma 348 G$_5$C$_7$ (ATCC number HB9916) has been made at the ATCC on November 23, 1988.

## Claims

1. A monoclonal antibody or antigen binding fragment thereof characterized in that :
   - it recognizes $\alpha$ and $\beta$ heavy chains of human atrial and ventricular myosin and $\beta$ heavy chains of slow human skeletal muscle myosin, and in a labelled form,
   - it is capable of producing an image of both ventricular and atrial damage resulting from myocardial cell necrosis, by localizing only in the cardiac necrotic tissue, obtainable from hybrids generated by fusing non secreting mouse myeloma cells with spleen cells from a mouse immunized with human ventricular myosin, then with the heavy chains of ventricular myosin, and screening in ELISA clones whose supernatants were positive for heavy chains of human cardiac myosin and human skeletal myosin, and whose supernatants do not cross react in ELISA with myosin light chains.

2. The monoclonal antibody or antigen binding fragment thereof according to claim 1 such as produced by hybridoma strain having the accession number ATCC HB 9916.

3. A composition suitable for use in imaging comprising a monoclonal antibody or antigen binding fragment according to anyone of claims 1 or 2 wherein said antibody is radiolabelled.

4. Use of the monoclonal antibody or antigen binding fragment according to anyone of claims 1 or 2 for the manufacture of a composition for the diagnosis and localization of myocardial or skeletal infarction.

5. The hybridoma strain having the accession number ATCC HB 9916.

6. A process for obtaining hybridoma strains capable of producing anti-$\alpha$ and anti-$\beta$ heavy chains monoclonal antibodies according to claim 1 which comprises
   - fusing non secreting mouse myeloma cells with spleen cells from a mouse immunized with human ventricular myosin, then with the heavy chains of ventricular myosin,
   - screening in ELISA the hybrids against heavy chains of cardiac myosin and human skeletal myosin, and
   - selecting the clones whose supernatants were positive for both antigens, and
   - further selecting the clones whose supernatants do not cross react with myosin light chains.

## Patentansprüche

1. Monoklonaler Antikörper oder Antigen-bindendes Fragment hievon, dadurch gekennzeichnet, daß:
   - er bzw. es die $\alpha$- und $\beta$-Schweren Ketten von Human-Atrium- und Ventrikel-Myosin sowie die $\beta$-Schweren Ketten von Myosin aus Human-Skelettmuskelzellen der langsamen Art erkennt und daß er bzw. es, in markierter Form,
   - befähigt ist, ein Bild sowohl von einer Ventrikel-Schädigung als auch von einer Atrium-Schädigung zu erzeugen, welche das Ergebnis einer Myokardzellnekrose ist, und zwar dadurch, daß er bzw. es sich nur in dem nekrotischen Herzgewebe lokalisiert, und daß er bzw. es aus Hybridzellen gewonnen werden kann, die durch Fusionieren nicht-sezernierender Mäuse-Myelomzellen mit Milzzellen aus einer Maus erzeugt worden sind, wobei diese Maus mit Human-Ventrikel-Myosin und dann mit den Schweren Ketten von Ventrikel-Myosin immunisiert worden ist, wonach in ELISA-Tests auf Klone durchgeprüft worden ist, deren Überstände hinsichtlich der Schweren Ketten von Human-Herzmuskel-Myosin und von Human-Skelettmuskel-Myosin positiv waren, und deren Überstände in einem ELISA-Test mit den Leichten Ketten von Myosin nicht kreuzreagierten.

2. Monoklonaler Antikörper oder Antigen-bindendes Fragment hievon nach Anspruch 1, von solcher Art, wie sie von dem Hybridomenstamm mit der Hinterlegungsnummer ATCC HB 9916 erzeugt werden.

3. Zusammensetzung, welche für die Verwendung zur Bildgebung geeignet ist und welche einen monoklonalen Antikörper oder ein Antigen-bindendes Fragment hievon nach einem der Ansprüche 1 oder 2 enthält, wobei dieser Antikörper radioaktiv markiert ist.

4. Verwendung des monoklonalen Antikörpers oder des Antigenbindenden Fragmentes hievon nach einem der Ansprüche 1 oder 2 zur Erzeugung einer Zusammensetzung für die Diagnose und Lokalisierung von Myokard- oder Skelettmuskelinfarkten.

5. Hybridomenstamm mit der Hinterlegungsnummer ATCC HB 9916.

6. Verfahren zur Gewinnung von Hybridomenstämmen, welche befähigt sind, monoklonale Anti-$\alpha$- und Anti-$\beta$-Schwere-Ketten-Antikörper nach Anspruch 1 zu erzeugen, welches Verfahren die folgenden Stufen umfaßt:
   - Fusionieren nicht-sezernierender Mäuse-Myelomzellen mit Milzzellen aus einer Maus, welche mit Human-Ventrikel-Myosin und dann mit den Schweren Ketten von Ventrikel-Myosin immunisiert worden ist,
   - Durchprüfen der Hybridzellen in ELISA-Tests gegen die Schweren Ketten von Herzmuskel-Myosin und von Human-Skelettmuskel-Myosin und
   - Auswählen derjenigen Klone, deren Überstände hinsichtlich beider Antigene positiv waren, und
   - weiteres Auswählen derjenigen Klone, deren Überstände mit den Leichten Ketten des Myosins nicht kreuzreagieren.

**Revendications**

1. Anticorps monoclonal ou fragment de celui-ci capable de se lier à un antigène caractérisé en ce que :
   - il reconnaît les chaînes lourdes $\alpha$ et $\beta$ de la myosine auriculaire et ventriculaire humaine et les chaînes lourdes $\beta$ des muscles squelettiques humains à contraction lente, et sous forme marquée,
   - il est capable de donner une image à la fois des lésions ventriculaires et auriculaires occasionnées par une nécrose des cellules du myocarde, en se localisant uniquement dans le tissu cardiaque nécrosé,
   - il peut être obtenu à partir d'hybrides produits par fusion de cellules de myélome murin non sécrétrices avec des cellules spléniques issues d'une souris immunisée avec la myosine ventriculaire humaine, puis avec les chaînes lourdes de la myosine ventriculaire, et criblage par ELISA des clones dont les surnageants donnent une réaction positive avec les chaînes lourdes de la myosine cardiaque humaine et de la myosine squelettique humaine, et dont les surnageants ne présentent pas de réaction croisée avec les chaînes légères de la myosine par ELISA.

2. Anticorps monoclonal ou fragment de celui-ci capable de se lier à un antigène selon la revendication 1, tel que produit par une souche d'hybridome portant le n° d'accès ATCC HB 9916.

3. Composition appropriée pour une utilisation en imagerie comprenant un anticorps monoclonal ou un fragment de celui-ci capable de se lier à un antigène selon l'une quelconque des revendications 1 ou 2, dans laquelle ledit anticorps est radiomarqué.

4. Utilisation de l'anticorps monoclonal ou d'un fragment de celui-ci capable de se lier à un antigène selon l'une quelconque des revendications 1 ou 2 pour la préparation d'une composition pour le diagnostic et la localisation de l'infarctus du myocarde ou des muscles squelettiques.

**5.** Souche d'hybridome portant le n° d'accès ATCC HB 9916.

**6.** Procédé d'obtention de souches d'hybridomes capables de produire des anticorps monoclonaux dirigés contre les chaînes lourdes $\alpha$ et $\beta$ selon la revendication 1 qui comprend :

- la fusion de cellules de myélome murin non sécrétrices avec des cellules spléniques issues d'une souris immunisée avec la myosine ventriculaire humaine, puis avec les chaînes lourdes de la myosine ventriculaire,
- le criblage par ELISA des hybridomes dirigés contre les chaînes lourdes de la myosine cardiaque et de la myosine squelettique humaine, et
- la sélection des clones dont les surnageants donnent une réaction positive avec les deux antigènes, et
- la sélection ultérieure des clones dont les surnageants ne présentent pas de réaction croisée avec les chaînes légères de la myosine.

_FIG. 1

_FIG 2

19

FIG. 3

FIG. 4

FIG. 5

O.D. 450 nm

x — x HVHC
o — o HAHC
o — o SkM
* — * DCHC.

Ab ng/ml

FIG. 6

TISSUE UPTAKE OF RADIOACTIVITY

PKD X 10

A   AMAB IG G1 I-125
B   AMAB F(AB')2 I-125
C   AMAB F(AB')2 DTPA IN-111
D   AMAB FAB I-125
E   CONTROL F(AB')2 I-125
F   99MTC PYP

FIG. 7

TISSUE/BLOOD                    TISSUE UPTAKE OF RADIOACTIVITY

30

                                              A    AMAB IG G1 I-125
                                              B    AMAB F(AB')2 I-125
                                              C    AMAB F(AB')2 DTPA IN-111
                                              D    AMAB FAB I-125
                                              E    CONTROL F(AB')2 I-125
20                                            F    99MTC PYP

10

0
    ABCDEF  ABCDEF  ABCDEF  ABCDEF  ABCDEF  ABCDEF  ABCDEF  ABCDEF
    BLOOD   LUNGS   MUSCLE  HEART-1 HEART-2 HEART-3 HEART-4 HEART-5

FIG. 8

EP 0 389 376 B1

Scintiscan of a dog injected with AM F(ab')$_2$-DTPA- $^{111}$In (2 mCi. 400 µg)   The image was obtained 21 h after i.v. administration of the $^{111}$In preparation and 24 h after artery occlusion. The infarcted area of the heart is clearly delineated above the liver.

FIG. 9